# EUROPÄISCHE PATENTSCHRIFT

(11) **EP 1 110 085 B1**
(45) Veröffentlichungstag und Bekanntmachung des Hinweises auf die Patenterteilung: **01.12.2004**
(21) Anmeldenummer: 99939890.2
(22) Anmeldetag: 07.09.1999
(51) Int. Cl.: G01N 33/487

(54) **SYSTEM ZUR BLUTZUCKERBESTIMMUNG**
SYSTEM FOR BLOOD GLUCOSE DETERMINATION
SYSTEME POUR DETERMINER LE TAUX DE SUCRE SANGUIN

(30) Priorität: 08.09.1998 DE 19840965
(43) Veröffentlichungstag der Anmeldung: 27.06.2001
(60) Teilanmeldung: 04018834.4
(73) Patentinhaber: Disetronic Licensing AG, 3401 Burgdorf (CH)
(72) Erfinder: HAUETER, Ulrich, CH-3506 Grosshöchstetten (CH); IMHOF, Erich, CH-3427 Utzenstorf (CH)
(74) Vertreter: Schwabe - Sandmair - Marx
(86) Internationale Anmeldenummer: PCT/CH1999/000416
(87) Internationale Veröffentlichungsnummer: WO 2000/014533

(56) Entgegenhaltungen:
- EP-A- 0 777 123
- WO-A-95/28878
- WO-A-97/08544
- GB-A- 2 311 866
- US-A- 4 543 955
- US-A- 4 871 351

## Beschreibung

Die Erfindung betrifft ein System mit einem Sensor und einer Standard-Steckkarte für eine Schnittstelle eines Computers, wobei das System der Überwachung des Gesundheitszustands einer Person dient.

Der Überwachung des Gesundheitszustands von Menschen kommt zunehmende Bedeutung zu. Insbesondere gilt dies für die Durchführung von Therapien, die eine dosierte Verabreichung eines oder mehrerer Wirkstoffe in Abhängigkeit von dem gesundheitlichen Zustand der betreffenden Person erfordern. Als Beispiel sei die Diabetestherapie genannt, wo die Verabreichung von Insulin in Abstimmung mit der gemessenen bzw. durch Messung ermittelten Glukosekonzentration in einer Körperflüssigkeit der Person erfolgt.

Die Überwachungsverfahren gerade im Rahmen von Therapien erfordern Spezialgerät allein für den Zweck, Messdaten für einen behandelnden Arzt oder die zu behandelnde Person verfügbar zu machen. Die Geräte zur Überwachung sind komplex und teuer oder gestatten eine Überwachung nur in einem sehr eingeschränkten Ausmaß. In der Diabetestherapie, wo die Glukosekonzentration in einer Körperflüssigkeit, insbesondere der Blutzuckergehalt, maßgebend ist, wird mit bekannten Vorrichtungen zur Überwachung der Glukosekonzentration nur die aktuell gemessene Konzentration angezeigt. Zumindest sind die Auswerte- und Anzeigemöglichkeiten sehr eingeschränkt. Ein Benutzer, der sich das Insulin, oder in einer anderen Therapie den entsprechenden anderen Wirkstoff, selbst verabreicht, könnte die Verabreichung seinen persönlichen Bedürfnisse genauer anpassen, wenn ihm beispielsweise auch "historische" Daten zur Verfügung stünden. Er könnte seine körperlichen Aktivitäten, Essgewohnheiten und zu verabreichenden Wirkstoffdosen genauer aufeinander abstimmen.

Aus der EP 0 777 123 A2 ist eine in einer Armbanduhr vorgesehene Überwachungseinrichtung bekannt, welche zum Beispiel den Glukosepegel bestimmen kann, welcher in einer Blutprobe gemessen wird.

Die WO 95/28878 offenbart eine Infusionspumpe und eine Glukosesensoranordnung, wobei wie aus den Figuren 1 und 4 der WO95/28878 ersichtlich eine Infusionspumpe 14 und eine Sensoreinheit 16 mit einer in einer Armbanduhr 28 vorgesehenen Überwachungseinheit telemetrisch verbunden sind.

Die US 4,543,955 offenbart eine Sensoranordnung mit einem in einen Körper implantierbaren physiologischen Sensor zum Überwachen der Arbeitsweise einer in den Körper implantierten Vorrichtung.

Die US 4,871,351 offenbart ein implantierbares Infusionssystem, welches über Funk mit einer externen Steuereinheit verbunden ist.

Es ist daher eine Aufgabe der Erfindung, eine bequeme, preiswerte und dennoch umfassende Überwachung eines Gesundheitszustands einer Person zu ermöglichen.

Die Erfindung löst die Aufgabe durch ein System mit den in Anspruch 1 aufgeführte Merkmalen Eine Messwertaufnahme nimmt einen Messwert auf und macht ihm für eine Überwachung eines Gesundheitszustands einer Person verfügbar. Die Steckkarte ist für eine Schnittstelle eines herkömmlichen Computers ausgebildet. Bei dem genannten Messwert handelt es sich um einen Messwert, der mittels eines Sensors z.B. an der Person zur Ermittlung des Gesundheitszustands aufgenommen worden ist. Das System ist für den Anschluss an eine definierte Schnittstelle des Computers vorbereitet. Es kann somit nicht nur vom Computerhersteller, sondern auch nachträglich von unabhängigen Herstellern bereitgestellt werden.

Vorteilhafte Ausführungsformen sind in den Ansprüchen 2 und 3 definiert.

In einer besonders bevorzugten Verwendung handelt es sich bei dem erfindungsgemäßen System um ein System, das in der Überwachung der Glukosekonzentration in einer Körperflüssigkeit der Person, insbesondere in der Überwachung des Blutzuckergehalts, eingesetzt wird.

Somit können herkömmliche Computer für die Überwachung verfügbar gemacht werden, die im allgemeinen über eine weit höhere Leistungsfähigkeit, insbesondere in Bezug auf Rechenleistung und Darstellungsmöglichkeiten, verfügen als bislang in der Überwachung eingesetzte Spezialgeräte. Ein weiterer Vorteil ist, dass insbesondere in Therapieformen, in denen eine Person sich den entsprechenden Wirkstoff selbst verabreicht, diese Person auf einen im allgemeinen bereits verfügbaren Computer zurückgreifen kann, den sie für andere Zwecke bereits benutzt und an dessen Betrieb sie gewöhnt ist. Ebenso ist die Übergabe von Gesundheitsdaten vom erfindungsgemäßen System zu anderen, weitverbreiteten Computerprogrammen möglich. Der erfindungsgemäße System bzw. dessen Software für den Computer ist zu den üblichen Programmen, wie beispielsweise Graphik-, Text- und/oder Tabellenkalkulationsprogrammen, kompatibel.

Die Standard-Steckkarte ist in einen Standardsteckplatz eines Computers einsteckbar, beispielsweise in der Form der heutzutage üblichen Standard-Steckkarten für einen äußeren Port eines Computers. Dabei wird die im Computer vorhandene Kapazität genutzt. Es sind insbesondere die Prozessor- und Bildschirmkapazitäten, aber auch die bereits vorhandenen Möglichkeiten für den Anschluss von Perpheriegeräten, die durch die Erfindung erschlossen werden. Der Benutzer kann daher zur Überwachung des Gesundheitszustands auf die ihm bekannte Computerumgebung zurückgreifen, was der Akzeptanz der Überwachung zugute kommt. Vorzugsweise wird mit solch einer Steckkarte die entsprechende Installations- und Anwendungssoftware für die Überwachung mitgeliefert, wie dies bei nachrüstbaren Steckmodulen im allgemeinen üblich ist.

Die Erfindung findet besonders bevorzugt Verwendung bei einem Computer einer Vorrichtung zur Selbstverabreichung eines Produktfluids oder eines in Verbindung damit eingesetzten Computers. Die Vorrichtung umfasst eine Fluidführungseinrichtung, ein Fördermittel zur Förderung einer zu verabreichenden Produktfluiddosis, eine Schnittstelle für drahtlose Kommunikation, eine Steuerung für das Fördermittel und ein Kommunikations-Endgerät. Die Fluidführungseinrichtung, das Fördermittel und die genannte Schnittstelle sind Bestandteile eines Verabreichungsgeräts. Das Kommunikations-Endgerät seinerseits ist von diesem Gerät körperlich losgelöst, d.h. zumindest bei Benutzung der Vorrichtung sind diese beiden Geräte mechanisch nicht miteinander verbunden. Eine etwaige Verbindung bei der Lagerung oder dem Transport soll jedoch nicht ausgeschlossen sein. Das Kommunikations-Endgerät ist mit einer weiteren Schnittstelle für drahtlose Kommunikation und einer optischen Anzeige ausgestattet. Zwischen dem Verabreichungsgerät und dem Kommunikations-Endgerät findet eine Kommunikation drahtlos über diese beiden Schnittstellen statt. Das Verabreichungsgerät und das Kommunikations-Endgerät verbleiben bei dem Benutzer, der sich das Produktfluid in eigener Regie verabreicht.

Die Fluidführungseinrichtung umfasst ein Reservoir für das Produktfluid und die damit verbundenen und vom Fluid bei der Verabreichung durchströmten Teile des Verabreichungsgeräts bis hin zu einer Fluidaustrittsstelle. Im Falle eines am Körper oder an der Kleidung zu befestigenden Verabreichungsgeräts wird die Fluidaustrittsstelle von einer Einstechnadel gebildet.

In einer ebenfalls bevorzugten Mischform sind das Fördermittel, ein Notsteuerungsteil hierfür, eine Energiequelle und die Schnittstelle für drahtlose Kommunikation implantiert, und das Reservoir wird am Körper oder an der Kleidung befestigt, wobei die Fluidführungseinrichtung stromab vom Reservoir naturgemäß teilweise ebenfalls implantiert ist; das Fördermittel, vorzugsweise eine Mikropumpe, ist dabei vorzugsweise selbst Teil des Fluidführungssystems, was es im übrigen grundsätzlich in den anderen, bezüglich des Aufbaus des Verabreichungsgeräts alternativen Ausführungsformen ebenfalls sein kann. Vorteile der Aufteilung des Verabreichungsgeräts in körperexternes Reservoir und körperinternes Fördermittel ist, dass der externe Teil verkleinert oder das Reservoir vergrößert werden kann und dass der Ort der Ausschüttung des Produktfluids für die Therapie besonders günstig gewählt werden kann. Gleichzeitig wird das Gesundheitsrisiko, das mit einem implantierten Reservoir verbunden ist, vermieden.

Das Produktfluid ist vorzugsweise eine flüssige Wirkstofflösung, wie sie im Rahmen einer Therapie verabreicht wird, insbesondere Insulin.

Die drahtlose Kommunikation erfolgt vorzugsweise über Funk. Aber auch eine Ultraschall- oder Infrarotkommunikation kommt in Betracht.

Auf der Anzeige des Kommunikations-Engeräts wird wenigstens ein Betriebsparameter des Verabreichungsgeräts dargestellt. Eine sich das Produktfluid selbst verabreichende Person kann den Betrieb des Verabreichungsgeräts und damit die Verabreichung unmittelbar und bequem am Kommunikations-Endgerät überwachen. Der Benutzer braucht das Verabreichungsgerät nicht zum Ablesen einer Anzeige zu lösen, da er die ihn interessierenden Informationen auf der Anzeige des bereits losgelösten Kommunikations-Endgeräts unabhängig vom Ort der Anbringung des Verabreichungsgeräts ablesen kann. Das Verabreichungsgerät kann hierdurch für einen Verabreichungszyklus bis zum nächsten Befüllen des Reservoirs an jeder geeigneten Stelle an der Kleidung des Benutzers oder unmittelbar am Körper befestigt werden. Bereits hierdurch erhöht sich der Tragekomfort für den Benutzer. Das Verabreichungsgerät kann für andere Personen nicht sichtbar ständig unter der Kleidung, beispielsweise unter einem Pullover, getragen werden. Dies erhöht die Akzeptanz des Geräts. Das erleichterte Ablesen erhöht gleichzeitig die Sicherheit, u. a. da der Benutzer deshalb seine Anzeige öfter prüft. Anzeigemittel am Verabreichungsgerät können vorgesehen sein, sind aber nicht mehr erforderlich und vorzugsweise am Verabreichungsgerät nicht oder nur in einem reduzierten Umfang vorhanden.

Ein für den Benutzer interessanter Betriebsparameter des Verabreichungsgeräts ist die verabreichte Fluidmenge. Bei einer kontinuierlichen oder quasi kontinuierlichen Produktausschüttung wird die Fluidmenge vorzugsweise über der Zeit als Förderrate aufgetragen. Insbesondere werden die in diskreten Zeitintervallen geförderten Fluidmengen dargestellt, wobei die Zeitintervalle in Anpassung an die Therapie und gegebenenfalls auch individuell für einen Benutzer geeignet vorgegeben oder wählbar sind.

Die in einem bestimmten Zeitintervall verabreichte Fluidmenge wird vorzugsweise aus der Position des Fördermittels ermittelt. Durch Vergleich einer Position am Ende eines Zeitintervalls mit einer bekannten Ausgangsposition zu Beginn des Zeitintervalls wird die im jeweiligen Zeitintervall geförderte Fluidmenge errechnet, d. h. durch Differenzbildung und Skalierung auf das Fluidreservoir. Durch Speicherung von im Zeitverlauf der Verabreichung durchlaufenen Positionen oder ermittelten Fördermengen kann die verabreichte Fluidmenge über der Zeit dargestellt werden. Die Darstellung erfolgt vorzugsweise grafisch. Es kann zur Erhöhung der Verabreichungssicherheit auch die Darstellung einer Abweichung von einer vorgegebenen Sollposition vorgesehen sein, beispielsweise ebenfalls über den Zeitverlauf der Verabreichung. Falls eine unzulässig große Abweichung von einer vorgegebenen Sollposition festgestellt wird, ist die Darstellung eines Warnsignals von Vorteil, das dem Benutzer das Auftreten einer zu beachtenden Abweichung signalisiert oder sogar das Maß der Abweichung und zweckmäßigerweise auch den Zeitpunkt des Auftretens anzeigt. Auch kann die Darstellung des zeitlichen Verlaufs der Abweichung zwischen dem Fördersoll und der tatsächlich geförderten Fluidmenge zweckmäßig sein. Aus der Position des Fördermittels kann vorteilhafterweise auch die noch verfügbare Restmenge des Produktfluids ermittelt werden, da jeder Position eine bestimmte Restmenge unmittelbar zugeordnet ist, wenn sich das Fluidreservoir in Form und Größe nicht ändert, indem beispielsweise stets gleiche Ampullen eingesetzt werden, oder Änderungen automatisch erkannt oder am Kommunikations-Endgerät manuell eingegeben werden.

Die Position des Fördermittels kann eine Sollposition sein, die von einer Steuerung vorgegeben wird, die das Fördermittel positionsgenau steuert. Nach einer ebenfalls bevorzugten Ausführungsform wird die von einem Sensor aufgenommene Istposition des Fördermittels zur Ermittlung der Fördermenge und der weiteren daraus ableitbaren Betriebsparameter verwendet.

In einer bevozugten Ausführungsform wird das Fluidführungseinrichtung in Bezug auf eine Okklusion überwacht. Das Auftreten und vorzugsweise das Ausmaß einer Okklusion werden am Kommunikations-Endgerät angezeigt. Eine Okklusion kann zum totalen Ausfall der Förderung oder zu einer Förderung mit verringerter Förderrate führen. Nach Feststellung eines Totalausfalls wird das Fördermittel automatisch stillgesetzt, und der Totalausfall wird am Kommunikations-Endgerät in Form eines Alarmsignals angezeigt. Wird trotz Okklusion weitergefördert, ist die tatsächliche Förderrate also nur geringer als eine vorgegebene Förderrate und wird festgestellt, dass diese Betriebsstörung durch eine Okklusion im Fluidführungssystem hervorgerufen wird, so wird dies vorzugsweise auf der optischen Anzeige des Kommunikations-Endgeräts angezeigt, insbesondere durch ein grafisches Warnsymbol und gleichzeitig geeigneter Darstellung der aufgetretenen Abweichung. Wie im Falle einer Abweichung aufgrund einer Fehlförderung, für die das Fördermittel ursächlich ist, kann der Benutzer bei der nächsten sich bietenden Gelegenheit ganz gezielt die Fehlförderung kompensieren. Vorzugsweise wird die Okklusion im Falle einer totalen Okklusion als Ereignis in einem Speicher des Kommunikations-Endgeräts zusammen mit dem Zeitpunkt des Feststellens gespeichert. Im Falle einer Minderförderung, deren Ausmaß aufgrund der Position des Fördermittels ermittelt werden kann, wird die Minderförderung über ihrem zeitlichen Verlauf gespeichert. Es kann, wie vorstehend bereits ausgeführt, die Darstellung der Fördermengen oder der Förderrate absolut oder die Darstellung der Abweichung von der Sollförderung vorgesehen sein.

In bevorzugter Weiterentwicklung wird eine im Fluidführungssystem aufgetretene Leckage festgestellt, zum Kommunikations-Endgerät gesendet und dort in Form eines Alarmsignals angezeigt. Vorzugsweise wird die Leckage als Ereignis in einem Speicher des Kommunikations-Endgeräts zusammen mit dem Zeitpunkt des Feststellens gespeichert und steht so für eine spätere Auswertung des Verabreichungsverlaufs zur Verfügung.

Das Auftreten einer Okklusion wird vorzugsweise mittels eines Sensors detektiert, der eine Reaktionskraft misst, die von dem Fördermittel auf eine Lagerung des Fördermittels ausgeübt wird. Die gemessene Reaktionskraft wird ständig mit einem geeignet vorgegebenen Schwellwert verglichen, dessen Überschreitung auf eine Okklusion schließen lässt. Der gleiche Sensor kann auch zur Detektion eines Lecks verwendet werden. Die gemessene Reaktionskraft wird dann zusätzlich mit einem geeignet vorgegebenen Schwellwert verglichen, dessen Unterschreitung auf eine Leckage schließen lässt.

In einem Strömungsquerschnitt des Fluidführungssystems ist vorzugsweise ein Mittel zur Erzeugung eines definierten Druckabfalls angeordnet. Der Druckabfall ist so groß bemessen, dass die Kraft, die das Fördermittel zur Überwindung dieses Strömungswiderstands aufbringen muss, deutlich größer ist als die bei ordnungsgemäßer Funktion des Verabreichungsgeräts auftretenden sonstigen, die Reaktionskraft bestimmenden Kräfte. Hierdurch wird eine Okklusion und insbesondere eine Leckage besonders sicher festgestellt.

Den höchsten Tragekomfort bietet ein Kommunikations-Endgerät, das Fernanzeige und Fernbedienung zugleich ist. Durch die Fernbedienung kann der Benutzer sein Verabreichungsgerät nicht nur diskret überwachen, sondern auch bedienen, sollte dies beispielsweise in Restaurants oder zu sonstigen gesellschaftlichen Gelegenheiten erforderlich sein. Eingabemittel des Kommunikations-Endgeräts können beispielsweise Tasten, ein Touchscreen oder eine Spracheingabe mit integriertem Mikrofon sein; letztere kann auch zusätzlich zu einem handbetätigten Eingabemittel vorgesehen sein, um eine besonders bequeme, in allen Situationen unauffällige Eingabe zu ermöglichen.

Die zur Ermittlung des wenigstens einen Betriebsparameters verwendete physikalische Größe, vorzugsweise die Position des Fördermittels und/oder die von dem Fördermittel ausgeübte Reaktionskraft, wird einem Prozessor der Vorrichtung aufgegeben, der daraus den Betriebsparameter bildet. Ist der Prozessor Bestandteil des Verabreichungsgeräts, so wird der von ihm gebildete Betriebsparameter der Schnittstelle des Verabreichungsgeräts aufgegeben und von dort zur Schnittstelle des Kommunikations-Endgeräts gesendet. Bevorzugterweise ist solch ein Prozessor Bestandteil des Kommunikations-Endgeräts, und es wird die physikalische Größe gesendet.

Eine vom Benutzer beeinflussbare Steuerung für das Fördermittel kann Bestandteil des Verabreichungsgeräts sein. Der Benutzer kann eine Einstellung dieser Steuerung mittels eines Eingabemittels des Kommunikations-Endgeräts ändern, beispielsweise zur Verabreichung eines Sonderbolus.

Zum Beispiel ist eine beeinflussbare Steuerung, insbesondere eine programmierbare Steuerung, oder der gesamte variierbare Teil der Steuerung des Fördermittels jedoch Bestandteil des Kommunikations-Endgeräts. Lediglich ein unumgänglich unmittelbar bei dem Fördermittel anzuordnender Teil der Steuerung, gegebenenfalls lediglich ein drahtlos gesteuertes Leistungsteil des Fördermittels, beispielsweise in Form nur eines getakteten Unterbrechers der Energiezufuhr zum Fördermittel, ist im Verabreichungsgerät untergebracht.

Bevorzugterweise umfasst das Verabreichungsgerät bei Anordnung des variierbaren Steuerungsteils im Kommunikations-Endgerät eine Notsteuerung, die bei einem Ausfall der drahtlosen Kommunikation ein festes Verabreichungsprogramm automatisch und autark bis zu einem Abschalten weiterfährt. Die Notsteuerung kann zusätzlich zu dem drahtlos angesteuerten Minimalsteuerungsteil der Hauptsteuerung vorgesehen sein. Sie kann jedoch auch durch das bei ordnungsgemäßer Funktion drahtlos gesteuerte Steuerungsteil gebildet werden, das auf Notbetrieb umschaltet, wenn innerhalb eines vorgegebenen Zeitintervalls keine Steuerungssignale ankommen. Die Notsteuerung kann nach einem für alle Verabreichungsgeräte einer Baureihe gleichen Standardprogramm arbeiten oder auch die Fähigkeit der individuellen Einstellbarkeit, vorzugsweise durch Programmierung, aufweisen, die nach Einstellung des Benutzers ebenfalls drahtlos über die Schnittstelle des Verabreichungsgeräts mittels des Kommunikations-Endgeräts oder eines anderen geeigneten Geräts vorgenommen wird, üblicherweise von einem Arzt. Eine weitergehende Veränderbarkeit weist sie jedoch nicht auf, sie fährt vielmehr das einmal eingestellte Standard- oder Individual-Programm ab.

Das Verabreichungsgerät kann sehr einfach wasserdicht gebaut werden, da ein Gehäuse wenig Durchbrechungen aufweist, wie beispielsweise für Eingabemittel. Im Extremfall müssen nur der Bereich der Schnittstelle und der Austritt für die Fluidführungseinrichtung abgedichtet werden.

Mittels einer Auswerteeinrichtung wird ein aufgenommener Messwert ausgewertet, indem daraus ein entsprechender Körperwert ermittelt wird. Ein Ergebnis der Auswertung wird an einer weiteren oder vorzugsweise der bereits genannten Anzeige des Kommunikations-Endgeräts zur Anzeige gebracht. Bei Verwendung der erfindungsgemäßen Vorrichtung in der Insulinbehandlung handelt es sich vorzugsweise um eine Auswerteeinrichtung zum Ermitteln eines Blutzuckergehalts; vorzugsweise kann damit zusätzlich der Hormonspiegel und/oder eine relevante Körpertemperatur ermittelt werden. Grundsätzlich ist solch eine Auswerteeinrichtung jedoch nicht auf eine Auswertung von Blutzuckermesswerten bzw. der vorgenannten Messwerte und Messwertgruppen beschränkt. Im Rahmen anderer Therapien, bei denen nicht Insulin, sondern andere Wirkstoffe verabreicht werden, werden dem entsprechenden Verwendungsfall angepasst andere Messwerte aufgenommen und ausgewertet.

Der Messwert kann von einem Sensor drahtlos als Messignal gesendet werden. Die Auswerteeinrichtung umfasst eine Messwertaufnahme zum Empfang dieses Signals. Die Messwertaufnahme wird in dieser Ausführungsform durch die Schnittstelle des Kommunikations-Endgeräts gebildet oder als zusätzliches Empfangsteil ausgebildet. In einer bevorzugten anderen Ausführungsform wird ein Sensor, der mit der Probe in Berührung steht oder stand, mit seinem Kontaktbereich in die Messwertaufnahme eingeführt, die das Messignal des Sensors somit durch unmittelbaren Kontakt erhält. Die Messwertaufnahme ist in beiden Fällen das Bindeglied zwischen dem Sensor und dem Kommunikations-Endgerät oder einer anderen Plattform für die Auswerteeinrichtung.

In der Ausführung mit dem körperlich einzuführenden Sensor können der Sensor und die Auswerteeinrichtung aus der Insulintherapie bekannten Sensoren und Auswerteeinrichtungen entsprechen.

Die Messwertaufnahme ist erfindungsgemäß integrierter Bestandteil der Standard-Steckkarte für Computer, beispielsweise einer PCMCIA Karte, und deshalb mit üblichen Computern wie PCs, Notebooks und Computern im Taschenformat einfach durch Einstecken in einen dafür vorgesehenen Steckplatz des Computers verbindbar. Die Steckkarte kann als reine Messwertaufnahme, d. h. als reines Durchleitungsglied für die Messwerte des Sensors, ausgebildet sein. Die nachfolgende Aufbereitung der Messwerte bis zum Erhalt der Körperwerte kann durch Komponenten vorgenommen werden, die der Computer von Hause aus aufweist. In dieser Ausführung ist die Steckkarte nur ein Sensorport. In einer anderen bevorzugten Ausführung sind auch Mittel zur Aufbereitung der Messwerte integrierter Bestandteil der Steckkarte. Auf der Steckkarte kann ein Permanentspeicher zum Zwischenspeichern der Messwerte oder der bereits aufbereiteten Messwerte vorhanden sein. Die Steckkarte kann mit einer vorinstallierten oder installierbaren speziellen Anwendersoftware geliefert werden.

Die Steckkarte kann ein integriertes Probennahmeset mit mehreren Sensorelementen und Mitteln zum Entnehmen von Proben beinhalten. Alternativ kann solch ein Probennahmeset aber auch Bestandteil des Kommunikations-Endgeräts sein, insbesondere dann, wenn die Auswerteeinrichtung integrierter Bestandteil des Kommunikations-Endgeräts ist.

In einer bevorzugten Ausführungsform ist das Kommunikations-Endgerät ein Standardcomputer im Taschenformat, beispielsweise ein Hand-Held oder vorzugsweise ein Palm-Sized Computer. Die Schnittstelle des Kommunikations-Endgeräts zur drahtlosen Kommunikation mit dem Verabreichungsgerät wird durch eine Steckkarte, beispielsweise eine PCMCIA Karte, für einen Steckplatz des Computers gebildet. Die gleiche Steckkarte beinhaltet vorzugsweise die Messwertaufnahme, die mit Zusatzkomponenten auf der Steckkarte bis zu einer Auswerteeinrichtung ausgebaut sein kann, und bildet so gleichzeitig Kommunikationsschnittstelle und den Sensorport.

Die Integration einer Auswerteeinrichtung oder auch nur einer Messwertaufnahme in einem Modul, vorzugsweise in einem Steckmodul, insbesondere einer Steckkarte, ist mit Vorteil auch in Verbindung mit Verabreichungsgeräten verwendbar, die ein losgelöstes Kommunikation-Endgerät nicht aufweisen. Die Modullösung kann in jeder Therapieüberwachung mit Vorteil eingesetzt werden, um die im Vergleich zu bekannten Einrichtungen größeren Rechen- und Speicherkapazitäten und auch umfangreicheren Anzeigemöglichkeiten eines Computers nutzen zu können. Als Steckmodul kann sie insbesondere ein eigenständig handelbares Produkt sein.

Vorzugsweise erfolgt bereits eine Konfigurierung und/oder eine Diagnose des Verabreichungsgeräts herstellerseitig drahtlos mittels Computer. Insbesondere erleichtert die drahtlose Kommunikation die Durchführung von Qualitätskontrollen. Da hierfür keine Drahtverbindungen hergestellt werden müssen, können Konfigurierung und Diagnose ohne Produktionsunterbrechung sozusagen bei laufendem Band durchgeführt werden, falls eine Schnittstelle für die drahtlose Kommunikation an geeigneter Stelle neben dem Band angeordnet wird.

Ein bevorzugtes Ausführungsbeispiel und die Anwendung der Erfindung wird nachfolgend anhand von Figuren erläutert. Es zeigen:
- Figur 1: eine Vorrichtung zur Selbstverabreichung eines Produktfluids mit einem Kommunikations-Endgerät für drahtlose Kommunikation,
- Figur 2: eine Ansicht des Kommunikations-Endgeräts,
- Figur 3: ein Blockdiagramm des Kommunikations-Endgeräts,
- Figur 4: eine Touchsreen Anzeige und
- Figur 5: eine drahtlose Kommunikation zwischen dem Verabreichungsgerät und zwei alternativen Kommunikations-Endgeräten.

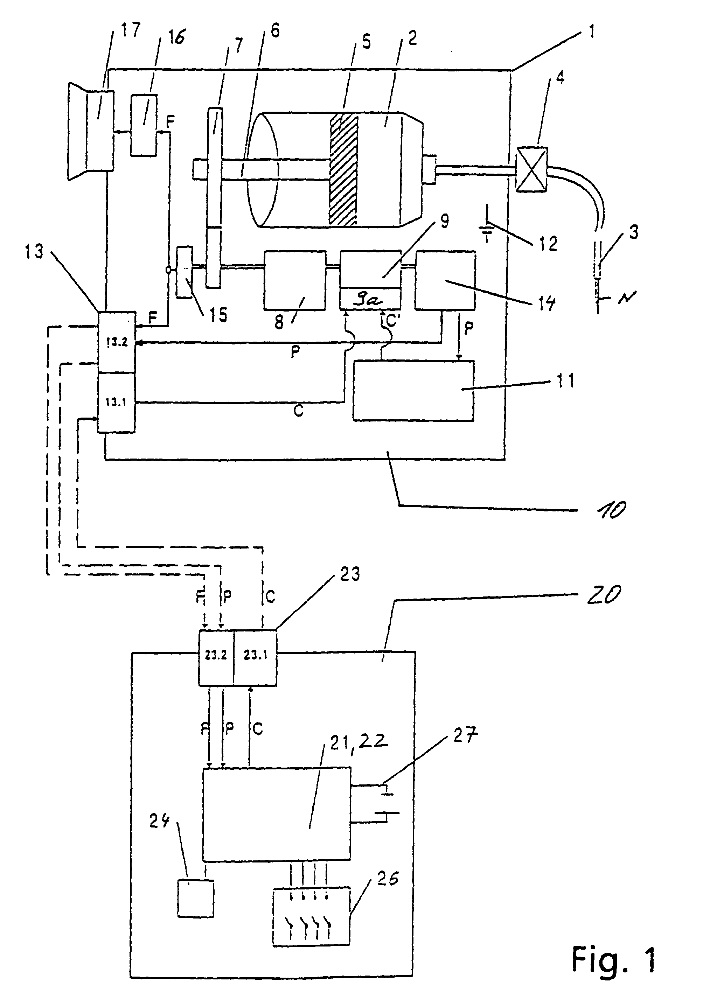

Figur 1 zeigt ein Verabreichungsgerät 10 in Form einer Infusionspumpe für Insulin, das drahtlos mit einem Kommunikations-Endgerät 20 kommuniziert.

Das Verabreichungsgerät 10 weist ein Gehäuse 1 auf, das mit geeigneten Haltemitteln versehen ist, so dass es von einem Benutzer an der Kleidung oder unmittelbar am Körper zum ständigen Tragen befestigt werden kann.

Das Insulin ist in einem Reservoir, das im Ausführungsbeispiel durch eine Ampulle 2 gebildet wird, enthalten. In der Ampulle 2 ist ein Kolben 5 verschiebbar aufgenommen. Das Verschieben des Kolbens 5 erfolgt mittels eines Spindeltriebs, dessen Abtriebsglied 6, im Ausführungsbeispiel eine Gewindestange, durch Drehantrieb eines Antriebsglieds 7, im Ausführungsbeispiel eine Gewindehülse, dem Gehäuse 1 gegenüber geradverschoben wird. Den Drehantrieb der Gewindehülse 7 bewirkt ein Schrittmotor 9, der über ein Getriebe 8 mit einem mit der Gewindehülse 7 kämmenden Stirnrad die Gewindehülse 7 drehantreibt. Ein Leistungsteil einer Steuerung für den Motor 9 ist mit 9a bezeichnet. Durch verdrehgesicherte Geradführung der Gewindestange 6 im Gehäuse 1 wird die Gewindestange 6 in axialer Richtung und gegen die Kolbenrückseite drückend verschoben. Der Kolben 5 wird unter der Einwirkung der Gewindestange 6 auf einen Ampullenauslass zu verschoben. Dadurch wird Insulin durch den Ampullenauslass verdrängt. An den Ampullenauslass angeschlossen ist eine Fluidleitung 3, an deren freien vorderen Ende eine Infusionsnadel N befestigt ist, die der Benutzer sich unter die Haut sticht und anschließend für die Selbstverabreichung des Insulins auf der Haut befestigt.

In der Fluidleitung 3 ist ebenfalls noch von dem Gehäuse 1 aufgenommen ein Ventil 4. angeordnet, das den Insulindurchfluss erst ermöglicht, wenn in der Ampulle 2 ein durch das Ventil 4 vorgegebener Mindestdruck überschritten wird.

Mit einem Positionssensor 14 wird ein Winkellagen-Istwert des Schrittmotors 9 gemessen und über Signalleitungen als Istposition P an eine Funkschnittstelle 13 und an eine im Gehäuse 1 untergebrachte Notsteuerung 11 für den Schrittmotor 9 weitergeleitet.

Die Notsteuerung 11 wird bei ordnungsgemäßer Funktion nur in einem Stand-by Modus betrieben. Die Notsteuerung 11 ist mittels Signalleitungen mit dem Positionssensor 14 und dem Leistungsteil 9a verbunden. Von dem Positionssensor 14 erhält sie die Istposition P und an das Leistungsteil 9a gibt sie ihr Stellsignal C' aus, falls die Notsteuerung 11 aktiviert ist, um den Motor 9 in eine nächste Sollposition zu fahren.
Als Energiequelle 12 für die energieverzehrenden Komponenten des Verabreichungsgeräts 10 dient eine im Gehäuse 1 untergebrachte elektrische Batterie.

Eine vom Kolben 5 auf das Gehäuse 1 ausgeübte Reaktionskraft F wird von einem Kraftsensor 15 ständig gemessen und in Form eines die gemessene Reaktionskraft F repräsentierenden Messignals ausgegeben. Die gemessene Reaktionskraft F wird über Signalleitungen der Funkschnittstelle 13 und einem Schwellwertvergleicher 16 zugeleitet, der bei Überschreitung eines vorgegebenen oberen Schwellwerts und bei Unterschreiten eines vorgegebenen unteren Schwellwerts für die gemessene Reaktionskraft F einen akustischen Alarm eines Alarmmittels 17 auslöst. Der Kraftsensor 15, der Vergleicher 16 und das Alarmmittel 17 bilden eine Einrichtung zum Auslösen eines Notalarms.

Der gesamte Antrieb des Kolbens 5, nämlich der Spindeltrieb 6, 7, das Getriebe 8, der Motor 9 mit Leistungsteil 9a, und auch der Positionssensor 14 sowie die Notsteuerung 11 sind gemeinsam auf einer im Gehäuse 1 in und gegen die Vorschubrichtung des Kolbens 5 verschiebbar geradgeführten Plattform gelagert. Die Lagerung der Plattform erfolgt in und gegen die Vorschubrichtung des Kolbens 5 frei schwimmend. Der Kraftsensor 15, beispielsweise ein DMS-Streifen in geeigneter Anordnung, ist an einer Unterseite der Plattform oder gegenüberliegend am Gehäuse 1 so angebracht, daß er bei einem Gegeneinanderdrücken der Plattform und des Gehäuses 1 ein Messignal ausgibt, das die dabei wirkende Kraft repräsentiert. Diese Kraft entspricht der vom Kolben 5 auf die Gewindestange 6 und somit auf die Plattform ausgeübten Reaktionskraft F.

Über die Funkschnittstelle 13 kommuniziert das Verabreichungsgerät 10 drahtlos mit dem Kommunikations-Endgerät 20, das im Ausführungsbeispiel als integriertes Fernanzeige- und Bediengerät ausgebildet ist. Das Kommunikations-Engerät 20 verfügt über eine Funkschnittstelle 23.

Beide Funkschnittstellen 13 und 23 weisen je sowohl einen Empfangsteil 13.1 bzw. 23.2 als auch einen Sendeteil 13.2 bzw. 23.1 auf. Über den Sendeteil 13.1 sendet das Verabreichungsgerät 10 ständig oder periodisch die Werte der gemessenen Reaktionskraft F und der Istposition P, die vom Empfangsteil 23.2 empfangen und einem Mikroprozessor 21 über Signalleitungen zugeführt werden.

Der Prozessor ermittelt aus der Istposition P insbesondere die geförderte Basalrate, vorzugsweise in Insulineinheiten pro Stunde IU/h, Bolusabgaben in Insulineinheiten IU und den Füllzustand der Ampulle 2 und/oder die aktuell verbleibende Restmenge IUR und/oder voraussichtliche Restförderdauer. Die ermittelte Basalrate und die Bolusabgaben werden gespeichert. Die Reaktionskraft F vergleicht er mit einem vorgegebenen oberen Schwellwert für diese Kraft. Bei Überschreiten des oberen Schwellwerts wird das Auftreten einer Okklusion im Fluidführungssystem festgestellt und mit dem Zeitpunkt ihres Auftretens gespeichert. Durch Vorgabe eines unteren Schwellwerts und Vergleich damit kann auch eine Leckage im Fluidführungssystem detektiert und der Zeitpunkt ihres Auftretens gespeichert werden.

Der Prozessor 21 bildet gleichzeitig eine veränderbare Steuerung 22 für den Motor 9 im Normalbetrieb, in dem die Notsteuerung 11 im Stand-by Modus verharrt. Nur im Notfall einer Kommunikationsstörung oder eines festgestellten sonstigen Steuerungsfehlers, der zum Verlust von Steuerungssignalen führt, übernimmt die Notsteuerung 11 anstelle der Prozessorsteuerung 22 die Steuerungsfunktion. Ansonsten steuert die Prozessorsteuerung 22 den Motor durch drahtlose Übermittlung ihrer Stellsignale C.

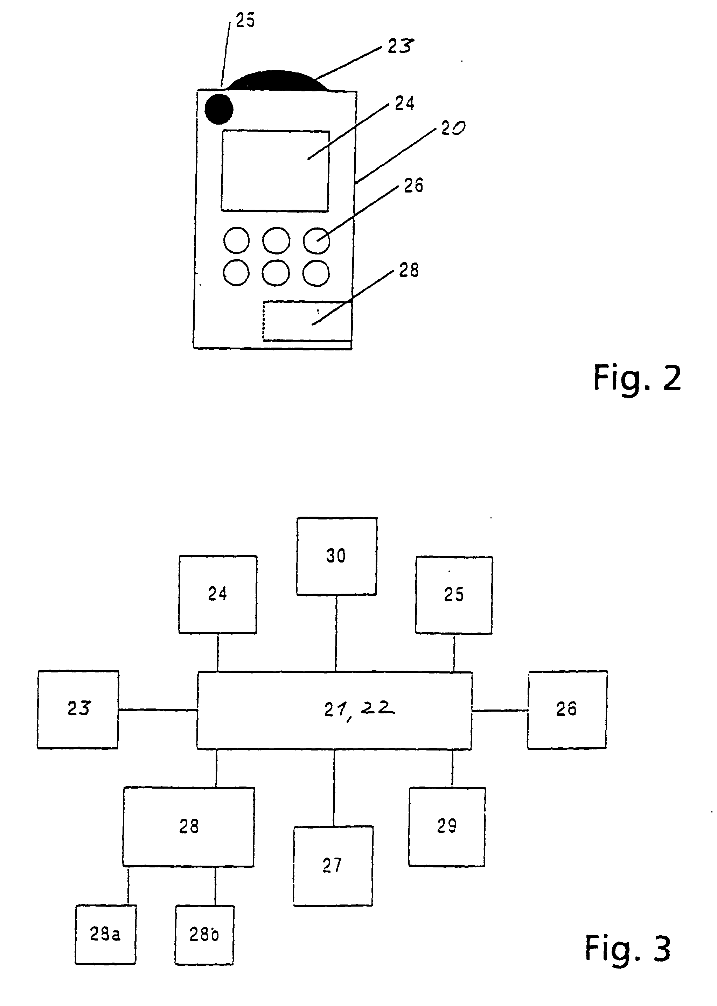

Figur 2 zeigt eine Vorderansicht des Kommunikations-Endgeräts 20. Sämtliche Komponenten des Kommunikations-Endgeräts 20 sind in einem leichten, in einer Hand haltbaren Gehäuse aufgenommen. Über die Schnittstelle 23 werden die Messignale P und F sowie Stellsignale C mit der Schnittstelle 13 ausgetauscht. Eine optische Anzeige 24 stellt für den Benutzer relevante Informationen dar. Dargestellte oder in einem Anzeigewahlmodus auf Abruf darstellbare Informationen sind zumindest Betriebsparameter des Verabreichungsgeräts 10. Die Ablesbarkeit wird insbesondere bei weiterlaufender Verabreichung durch die auch dabei bequem in der Hand haltbare Fernanzeige erheblich verbessert. Die Vielfalt und auch die Komplexität der angezeigten Information kann erhöht werden, ohne das Gewicht und die Abmesssungen des Verabreichungsgeräts vergrößern zu müssen.

Die Handhabbarkeit wird weiter noch dadurch verbessert, dass das Kommunikations-Endgerät 20 nicht nur als Fernanzeige, sondern auch als Fernbedienung ausgebildet ist. Hierzu weist das Endgerät 20 Eingabemittel 26 in Form von Tasten auf, die ein Einwirken auf den Prozessor 21 und auch auf die durch ihn gebildete Steuerung 22 ermöglichen. So kann durch Drücken auf eine der Tasten eine spontane oder eine in Abhängigkeit von der Eingabe zeitverzögert vorprogrammierte Bolusabgabe ausgelöst werden. Die optische Anzeige 24 und die Tasten 26 können auch zusammenwirken, indem auf der Anzeige 24 beispielsweise Tasteneinstellungen oder durch die Tasten 26 auswählbare Informationen angezeigt werden. Ferner weist das Kommunikations-Endgerät 20 ein akustisches Alarmmittel 25 auf, das gefährliche Fehlfunktionen des Kommunikations-Endgeräts 20 und auch des Verabreichungsgeräts 10 akustisch zur Kenntnis bringt.

Bezüglich der Bedienfunktion kann mittels des Endgeräts 20 das Verabreichungsgerät 10 ein- und ausgeschaltet und eine Bolusabgabe ausgelöst oder zumindest erhöht und erniedrigt werden. Ferner können die Basalrate und die temporäre Basalrate eingestellt und vorzugsweise auch verstellt werden. Auch die Befüllung des Katheters nach einem Ampullenwechsel kann derart kontrolliert erfolgen.

In Figur 3 sind die wesentlichen Komponenten des Kommunikations-Endgeräts 20 in Form eines Blockdiagramms miteinander verknüpft. Als zentraler Baustein steuert der Mikroprozessor 21 sowohl die optische Anzeige 24 als auch die akustische Anzeige 25 in Abhängigkeit von Eingangssignalen, die er von der Schnittstelle 23 oder dem Eingabemittel 26 erhält. Angedeutet ist auch eine Energiequelle 27.

Das Endgerät 20 ist mit einer Überwachungseinrichtung 29 zur Überwachung der Lage des Schrittmotors 9 ausgestattet. Hierzu erhält es die Istposition P des Lagesensors 14 über die Schnittstelle 23 zusammen mit dem Sollwert von der Prozessorsteuerung 22. Wird eine vorgegebene Höchstdifferenz dem Betrage nach überschritten, so wird durch das akustische Alarmmittel 25 ein Alarmsignal abgegeben. Noch tolerierbare Abweichungen werden angezeigt und können vom Benutzer über die Fernbedienung bei sich bietendem Anlass berücksichtigt und ausgeglichen werden, beispielsweise mit einer Sonderbolusabgabe. Eine Regelung erfolgt nicht. Der Soll/Ist-Vergleich, der lediglich der Sicherheit dient, wird bei einem Ausfall der Prozessorsteuerung 22 auch von der Notsteuerung 11 durchgeführt, wobei eine nicht tolerierbare Abweichung dann durch das Alarmmittel 17 zur Kenntnis gebracht werden kann.

Der Mikroprozessor 21 hat auf einen eigenen Speicher 30 des Kommunikations-Endgeräts 20 Zugriff, in dem insbesondere ein personenindividueller Setup und die historischen Daten der Verabreichung gespeichert sind bzw. ständig akkumulierend weitergespeichert werden. Auch Blutzuckermesswerte können über der Zeit gespeichert werden, entweder in einem eigenen Bereich des Speichers 30 oder in einem weiteren endgeräteeignen Speicher. So kann der Benutzer die Historie der Verabreichung mit den im Zeitverlauf zugeordneten, gespeicherten Blutzuckermesswerten vergleichen. Hierdurch kann der Benutzer für ihn wertvolle Rückschlüsse und gegebenenfalls Folgerungen für die zukünftige Verabreichung und individuelle Einstellung seines Verabreichungsgeräts ziehen. Das Kommunikations-Endgerät 20 ist das elektronische Tagebuch des Benutzers.

Eine Blutzuckermesseinrichtung ist im Gehäuse des Kommunikations-Endgeräts 20 ebenfalls integriert. Die Blutzuckermesseinrichtung weist eine Messwertaufnahme 28b auf. Ein Sensor 28a misst den Blutzuckergehalt einer Blutprobe und/oder Zellflüssigkeitsprobe. Die Messwertaufnahme 28b übernimmt ein vom Sensor 28a ausgegebenes Messignal, dessen Größe vom Blutzuckergehalt der Probe abhängt, und führt es dem Mikroprozessor 21, d.h. einer vom Mikroprozessor gebildeten Auswerteeinrichtung 28, zu. Der von dem Prozessor daraus gewonnene Messwert wird im Speicher 30 gespeichert, um so auch für eine spätere Darstellung an der Anzeige 24 verfügbar zu sein. Bei dem Sensor 28a handelt es sich um einen üblichen Sensor, insbesondere in Form eines Plättchens, mit einem Probenbereich zum Aufbringen der Probe und einem Kontaktbereich zum Einführen und zur Kontaktherstellung in und mit der Messwertaufnahme 28b. Die Blutzuckermesseinrichtung besteht aus der Messwertaufnahme 28b als Aufnahme- und Kontakteinrichtung für den Sensor 28a, einem Anschluss zum Verbinden mit dem Prozessor 21 und dem Prozessor 21 selbst, der anwendungsspezifisch programmiert die Auswertefunktion erfüllt und dem im Ausführungsbeispiel alle weiteren im Zusammenhang mit der Auswertung und Darstellung der Messignale des Sensors 28a stehenden Aufgaben zufallen.

Die Auswerteeinrichtung 28 kann durch einen Wandler gebildet werden, falls sie auf dem lösbaren Modul integriert ist, der das vom Sensor 28a übernommene Messsignal in ein vom Prozessor 21 lesbares Signal umwandelt. Die Auswerteeinrichtung 28 kann ein vom Sensor 28a übernommenes Messignal in einer bevorzugten weiteren Ausführungsform selbst in den Speicher 30 abspeichern, so dass der Prozessor 21 von der Aufgabe der Abspeicherung entlastet wird. Es ist auch möglich, die Ermittlung des Blutzuckergehalts in solch ein Messwertaufnahme- und Auswertemodul zu verlagern. Auch ein Speicher kann Bestandteil des Moduls sein, um die Sensormessignale darin zwischenspeichern zu können. Eine Messeinrichtung in der Grundform als reine Messwertaufnahme 28b oder in einer der vorgenannten Ausbaustufen kann mit Vorteil bei jeder Art einer Vorrichtung, insbesondere einer gattungsgemäßen Vorrichtung ohne Betriebsparameteranzeige, verwendet werden, beispielsweise zusammen mit einer reinen Fernbedienung oder einer Fernanzeige mit anderen angezeigten Informationen. In der Ausführungsform als Steckkarte kann sie in der vorgenannten Grundform oder in einer der vorgenannten Ausbaustufen mit Vorteil auch ein eigenständiges, von einer bestimmten Vorrichtung zur Verabreichung eines Produktfluids unabhängiges Produkt sein. Sie kann in dieser Form besonders bequem jede von einem Benutzer überwachte Therapie unterstützen. Sie kann eine eigene Anzeige aufweisen, um auch ohne Computer wie herkömmliche Auswerteeinrichtungen verwendbar zu sein.

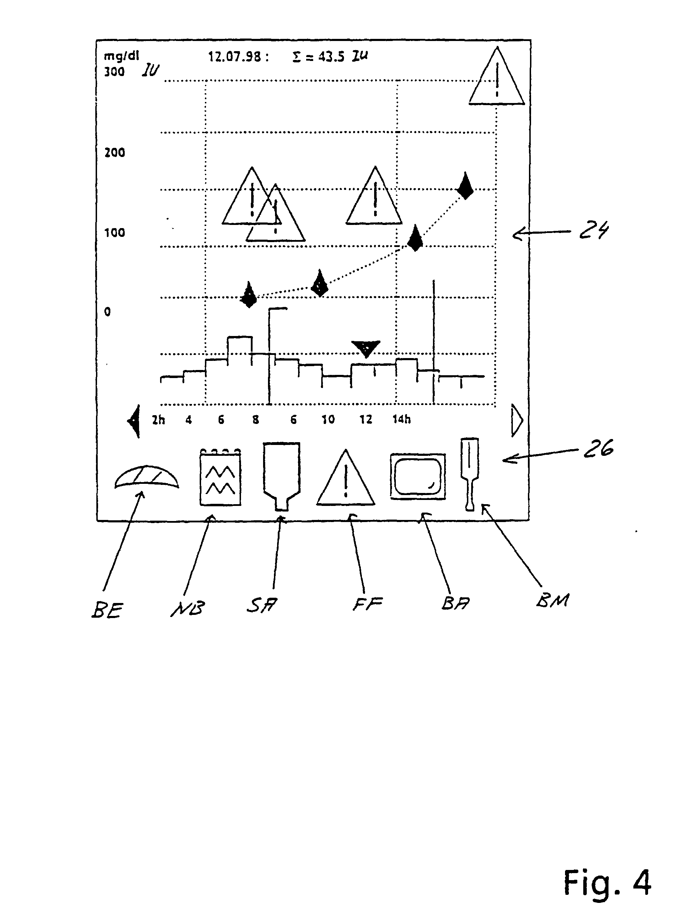

Figur 4 zeigt eine Darstellung, wie sie nach Anwählen eines geladenen Diabetesprogramms auf einem Touchscreen eines Palm Top Computers erscheint. Dessen Display ist Grafikanzeige und Eingabemittel zugleich.

Permanent dargestellt wird die im Verlaufe der letzten Stunden akkumuliert pro Stunde als Basalrate verabreichte Insulinmenge in Form eines Säulendiagramms in Insulineinheiten pro Stunde. Auch Sonderbolusabgaben, veranlasst durch den Benutzer, werden permanent dargestellt. Dabei steht ein einfacher senkrechter Strich für einen normalen Bolus und ein oben abgewinkelter Strich für einen verlängerten Bolus. Die mit dem Bolus verabreichte Menge wird durch die senkrechte Strichlänge angezeigt. Auf der Zeitachse ist der Zeitraum der letzten Stunden, beispielsweise der letzten vierundzwanzig Stunden, mit Uhrzeitangabe aufgetragen. Die verabreichten Insulinmengen werden vom Prozessor 21 aus den Istpositionen P ermittelt und im Speicher 30 abgelegt. Sie stehen so jederzeit für Darstellungs- und sonstige Auswertungszwecke zur Verfügung. Solange die Überwachungseinrichtung 29 keine Fehlfunktion feststellt, können statt der Istpositionen P auch einfach die Sollpositionen von der Steuerung 21 übernommen und zur Ermittlung der geförderten Insulinmengen verwendet werden. Schließlich wird die in den letzten vierundzwanzig Stunden vor dem Ablesen verabreichte Insulingesamtmenge in Form ihres numerischen Werts hinter einem Summenzeichen permanent angezeigt.

Unterhalb des Anzeigebereichs weist das Display einen Eingabebereich mit Eingabefeldern 26 auf. Jedes der Eingabefelder 26 ist mit einem die jeweilige Funktion darstellenden grafischen Symbol belegt. Von links nach rechts haben die Eingabefelder 26 die Bedeutung: Broteinheiten BE, Notizbuch NB, Ampullenstatus SA, Fehlfunktionen FF, Blutzuckeranzeige BA und Blutzuckermessung BM. Durch Druck auf eines der Felder wird dessen Funktion aktiviert, durch nochmaliges Drücken desaktiviert. Es kann jede beliebige Kombination von Feldern gleichzeitig aktiv sein. Figur 4 zeigt das Display in einem Zustand, in dem nur die Felder FF und BA akiviert sind.

Durch Druck auf das Feld BE wird die manuelle Eingabe der aufgenommenen Broteinheiten mit Zeit- und Mengenangabe möglich. Durch Druck auf das Feld NB kann der Benutzer ihm wichtig erscheinende persönliche Notizen eingeben, die mit dem vom Benutzer eingegebenen Zeitpunkt abgespeichert werden oder, falls der Benutzer die Zeit nicht definiert, automatisch mit dem Zeitpunkt der Aktivierung des Felds NB. Gleichzeitig werden auf dem Display dem betätigten Eingabefeld entsprechend die für den dargestellten Zeitraum eingegebenen Broteinheiten oder Notizen zeitecht dargestellt. Bei Drücken des Feldes SA wird der aktuelle Füllzustand der Ampulle in Prozent der Füllmenge einer neuen Ampulle und/oder als noch verbleibende Restmenge Insulin oder als geschätzte Restförderdauer angezeigt. Ebenso kann zusätzlich der Zeitpunkt des letzten Ampullenwechsels angezeigt werden. Bei Betätigung des Felds FF wird eine Abweichung vom Sollbetrieb, beispielsweise eine Okklusion, eine Kommunikationsstörung oder ein elektrischer Ausfall, zeitecht innerhalb des Warnsymbols geeignet dargestellt. Drücken des Feldes BA bewirkt die Darstellung des aus Messungen gewonnenen Blutzuckergehalts in beispielsweise Milligramm Zucker pro Deziliter Blut zum Zeitpunkt der jeweiligen Messung. Diesen Zeitpunkt bestimmt der Benutzer durch Drücken des Felds BM. Denkbar wäre jedoch auch die automatische Bestimmung dieses Zeitpunkts.

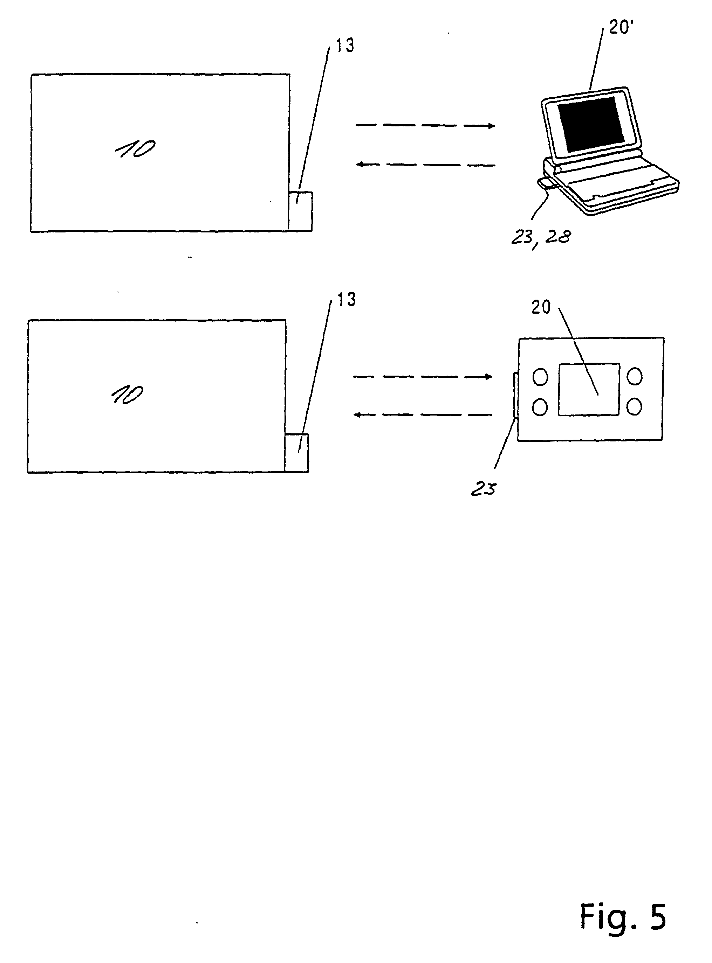

In Figur 5 ist neben der Möglichkeit der drahtlosen Kommunikation mit dem Endgerät 20 auch die Möglichkeit der Kommunikation über die gleiche Schnittstelle 13 mit einem Computer 20' dargestellt, in Figur 5 ein Notebook. Die Verwendung eines mit einem vergleichsweise großen Bildschirm ausgerüsteten Standard Computers 20' erhöht die Vielfalt und Komplexität der Verwendbarkeit der Fernanzeige und Fernbedienung erheblich. Mittels des PC's 20' kann das Verabreichungsgerät bis auf die personenindividuellen Einstellungen vollständig herstellerseitig konfiguriert werden, vorzugsweise durch Programmierung. Auch die individuelle Konfigurierbarkeit beim Benutzer vor Ort und insbesondere die Auswertemöglichkeiten des Benutzers, beispielsweise der Vergleich der Verabreichungshistorie mit den Blutzuckerwerten, kann besonders komfortabel und umfangreich gestaltet werden. Auch beim Hersteller kann die drahtlose Kommunikation zur wirtschaftlichen Konfigurierung und/oder Qualitätskontrolle eingesetzt werden. So muss insbesondere zur Qualitätskontrolle nicht jeweils eine Drahtverbindung zwischen dem zu kontrollierenden Verabreichungsgerät und dem zu Kontrollzwecken eingesetzten Computer hergestellt werden. Die Qualitätskontrolle kann mittels der drahtlosen Kommunikation sogar im Vorbeifördern der Verabreichungsgeräte durchgeführt werden.

## Patentansprüche

1. System umfassend:
- einen Sensor (28a), und
- eine Standard-Steckkarte für einen Computer, wobei die Steckkarte ausfweist: eine Blutzuckermesseinrichtung, umfassend eine Messwertaufnahme (28b), welche ein von dem Sensor (28a) ausgegebenes Messsignal übernimmt und eine Einrichtung (28), die mit der Messwertaufnahme (28b) verbunden ist und den von dem Sensor (28a) gemessenen Blutzuckergehalt in ein von dem Computer verarbeitbares Signal umwandelt.

2. System nach Anspruch 1 mit einem Speicher (30) auf der Steckkarte zur Speicherung des von der Auswerteeinrichtung (28) umgewandelten Signals.

3. Vorrichtung zur Selbstverabreichung eines Produktfluids umfassend ein System nach einem der vorhergehenden Ansprüche.

## Claims

1. A system including a sensor (28a) and a standard plug-in board or card for a computer, wherein said plug-in board or card comprises: a blood sugar measuring means including a transducer (28b) which receives a measurement signal outputted from the sensor (28a) and a means (28) which is connected to the transducer (28b) and converts the blood sugar content measured by the sensor (28a) into a signal that can be processed by the computer.

2. The system as set forth in claim 1, comprising a memory (30) for storing the signal converted by the evaluation means (28).

3. A device for self-administration of a fluid product, including a system as set forth in any one of the preceding claims.

## Revendications

1. Système comprenant:
- un détecteur (28a), et
- une carte enfichable standard pour un ordinateur, la carte enfichable présentant:
un dispositif de mesure de glycémie comprenant un système de transmission de valeurs de mesure (28b) qui reçoit un signal de mesure émis par le détecteur (28a) et un dispositif (28) qui est relié au système de transmission de valeurs de mesure (28b) et convertit la glycémie mesurée par le détecteur (28a) en un signal utilisable par l'ordinateur.

2. Système suivant la revendication 1 avec une mémoire (30) sur la carte enfichable pour la mémorisation du signal converti par le dispositif d'évaluation (28).

3. Dispositif d'auto-administration d'un fluide de produit comprenant un système suivant l'une des revendications précédentes.
